# EUROPÄISCHE PATENTANMELDUNG

(11) **EP 2 138 499 A1**
(43) Veröffentlichungstag der Anmeldung: **30.12.2009**
(21) Anmeldenummer: 09450110.3
(22) Anmeldetag: 04.06.2009
(51) Int. Cl.: C07D 491/04

(54) **Verfahren zum Herstellen von hochreinen Benzazepinderivaten**

(30) Priorität: 26.06.2008 AT 10212008
(71) Anmelder: Sanochemia Pharmazeutika AG, 1090 Wien (AT)
(72) Erfinder: Gerdes, Klaus, 40233 Düsseldorf (DE); Gungl, József, 9400 Sopron (HU); Kälz, Beate, 7035 Steinbrunn (AT); Rothenburger, Jan, 7064 Oslip (AT); Welzig, Stefan, 1030 Wien (AT)
(74) Vertreter: Dungler, Karin

(57) **Zusammenfassung**

Die Erfindung betrifft ein Verfahren zur Herstellung von hochreinem Galanthamin bzw. hochreinen Galanthaminderivaten, wobei man von racemischem Bromnarwedin ausgeht, welches unter Palladiumkatalyse debromiert wird. Erfindungswesentlich ist dabei die Aufarbeitung des Reaktionsgemisches, welche in Gegenwart von Sauerstoff oder Peroxiden erfolgt, sodass der Palladiumkatalysator in eine unlösliche Form, leicht abtrennbare Form übergeführt wird. Die weitere Reaktion erfolgt durch Reduktion von enantiomerenreinem Narwedin zu enantiomerenreinem Galanthamin, wobei anschließend alkyliert bzw. dealkyliert wird, sodass eine entsprechende Substitution am Ring-Stickstoffatom erzielt wird. Durch weitere Reinigung, wie Umkristallisieren, werden Restanteile von Palladium unterhalb von 5 ppm erzielt, sodass die direkte Verwendung als pharmazeutischer Rohstoff ermöglicht wird.

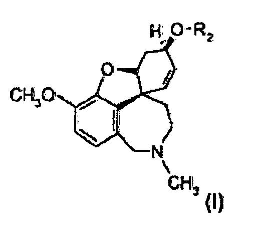

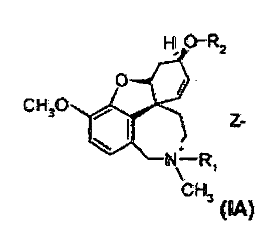

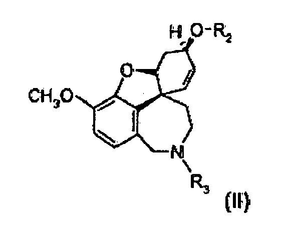

## Beschreibung

Die Erfindung betrifft Verfahren zum Herstellen von hochreinem 4a,5,9,10,11,12,-Hexahydro-6H-benzofuro[3a,3,2-ef][2]benzazepinderivaten der allgemeinen Formel I 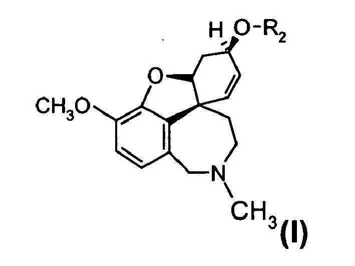 der Formel IA 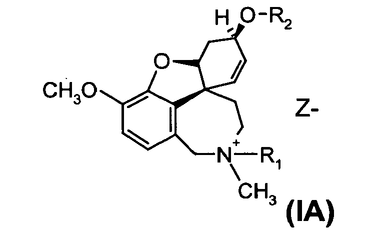 und der Formel II 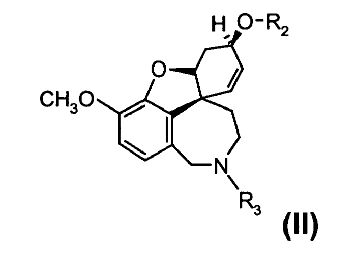 worin R1 ausgewählt ist aus der Gruppe bestehend aus Wasserstoff, Hydroxy, Alkoxy, niedriges Alkyl (C2-C10), welches gegebenenfalls durch wenigstens ein Halogen substituiert ist, niedriges Alkenyl (C2-C10), Aryl, Aralkyl, Aryloxyalkyl, R2 ausgewählt ist aus der Gruppe bestehend aus niedriges Alky (C2-C10)I, welches gegebenenfalls durch wenigstens ein Halogen substituiert ist, niedriges Alkenyl (C2-C10), niedriges Alkinyl (C2-C10), Aryl, Aralkyl, Aryloxyalkyl, Alkylcarbonyl, Arylcarbonyl, Aralkylcarbonyl, Alkyloxycarbonyl, Aryloxycarbonyl, Aralkyloxycarbonyl, Alkylthionyl, Arylthionyl, Aralkylthionyl, Alkyloxythionyl, Aryloxythionyl, Aralkyloxythionyl, Alkylsulfonyl, Aralkylsulfonyl, Arylsulfonyl, Carboxamid, Thiocarboxamid, R3 ausgewählt ist aus der Gruppe bestehend aus Wasserstoff, Hydroxy, Alkoxy, niedriges Alkyl (C2-C10), welches gegebenenfalls durch wenigstens ein Halogen substituiert ist, niedriges Alkenyl (C2-C10), niedriges Alkinyl (C2-C10), Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl und wobei Z-ein Anion einer pharmazeutisch annehmbaren organischen Säure oder ein anorganisches Anion ist.

Galanthamin ist ein vorwiegend in Pflanzen vom Typus Amaryllidaceae vorkommendes Alkaloid mit hoher pharmakologischer Aktivität. Hervorzuheben ist insbesondere seine Wirkung als selektiver Acetylcholinesterase Inhibitor und die damit in Zusammenhang stehende Anwendung bei Behandlung neurodegenerativer Erkrankungen, wie die Alzheimer'sche Erkrankung. Die aus dem natürlich vorkommenden kaukasischen Schneeglöckchen Galanthus Woronoyi isolierten Mengen sind jedoch nicht ausreichend, um den Bedarf eines pharmazeutischen Rohstoffes abzudecken. Seit Ende der sechziger Jahre sind daher Galanthaminsynthesen bekannt, welche aber mitunter lange und unwirtschaftliche Reaktionswege mit schlechten Gesamtausbeuten zeigen.

Gemäß der WO-A-97/110777 soll durch gezielte Auswahl von Bromnarwedin als Ausgangsprodukt ein wirtschaftlicher Weg für die Galanthaminsynthese insofern geschaffen werden, als Bromnarwedin mit Palladium (II) Acetat unter Zusatz von Triphenylphosphin debromiert wird. Das erhaltene racemische Narwedin enthält allerdings etwa 700 - 800 ppm Palladium, welches auch nach mehrmaliger Behandlung mit Aktivkohle nicht abgetrennt werden kann. Auch bei weiteren Reaktionsschritten, wie die Reduktion von racemischem Narwedin, welche gemäß der WO-A-96/12692 der Anmelderin beschrieben wird, wird Palladium trotz mehrmaliger Aufarbeitung weiter im Reaktionsendprodukt nachgewiesen. Galanthamin bzw. Galanthaminderivate, welche Palladium in einem Ausmaß von 700 - 800 ppm aufweisen, sind jedoch für die Herstellung von Arzneimitteln, wie Mittel zur Behandlung der Alzheimer'schen Erkrankung nicht geeignet, da im Organismus, bedingt durch die Palladiumreste, unerwünschte Nebenwirkungen auftreten können. Demgemäß sind Grenzwerte mit <5 ppm für die orale Applikation von Arzneimitteln normiert, siehe "Note for Guidance on specification limits for residues of metal catalysts" CPMP/SWP/QWP/4446/00..

Der Erfindung liegt daher die Aufgabe zugrunde, Verfahren der eingangs genannten Art anzugeben, mit welchen die vorgenannten, normierten Grenzwerte für Galanthaminderivate der Formel I eingehalten werden können.

Erfindungsgemäß wird ein Verfahren zur Herstellung der eingangs genannten Verbindungen mit der allgemeinen Formel (I) vorgeschlagen, wobei man in einem Reaktionsschritt 1 racemisches Bromnarwedin (III) mit Palladium (II) Acetat und Triphenylphosphin debromiert, in einem Reaktionsschritt 2 das Reaktionsgemisch, beinhaltend racemisches Narwedin (IV) unter Sauerstoffkontakt bzw. Zugabe von Peroxiden aufarbeitet und in einem zum enantiomerenreinem Narwedin (V) umwandelt und wobei man in einem Reaktionsschritt 3 durch Reduktion enantiomerenreines Galanthamin der allgemeinen Formel (VI) erhält und in einem Reaktionsschritt 4 durch O-Alkylierung Verbindungen der allgemeinen Formel (I) bzw. in einem Reaktionsschritt 4' durch O-Alyklierung sowie anschließender Salzbildung Verbindungen der allgemeinen Formel (IA) erhält bzw. in einem Reaktionsschritt 4" durch O-Alyklierung, N-Demethylierung und N-Alkylierung Verbindungen der allgemeinen Formel (II) erhält.

Alternativ wird erfindungsgemäß ein Verfahren zur Herstellung der eingangs genannten Verbindungen mit der allgemeinen Formel (I) bzw. (II) vorgeschlagen, wobei man in einem Reaktionsschritt 1 racemisches Bromnarwedin (III) mit Palladium (II) Acetat und Triphenylphosphin debromiert, in einem Reaktionsschritt 2 das Reaktionsgemisch, beinhaltend racemisches Narwedin (IV) unter Verwendung von Peroxiden aufarbeitet und in einem zum enantiomerenreinem Narwedin (V) umwandelt und wobei man in einem Reaktionsschritt 3 durch Reduktion enantiomerenreines Galanthamin der Formel (VI) erhält und in einem Reaktionsschritt 4 durch O-Alkylierung Verbindungen der allgemeinen Formel (I) bzw. in einem Reaktionsschritt 4' durch O-Alyklierung sowie anschließender Salzbildung Verbindungen der allgemeinen Formel (IA) erhält bzw. in einem Reaktionsschritt 4" durch O-Alyklierung, N-Demethylierung und N-Alkylierung Verbindungen der allgemeinen Formel (II) erhält.

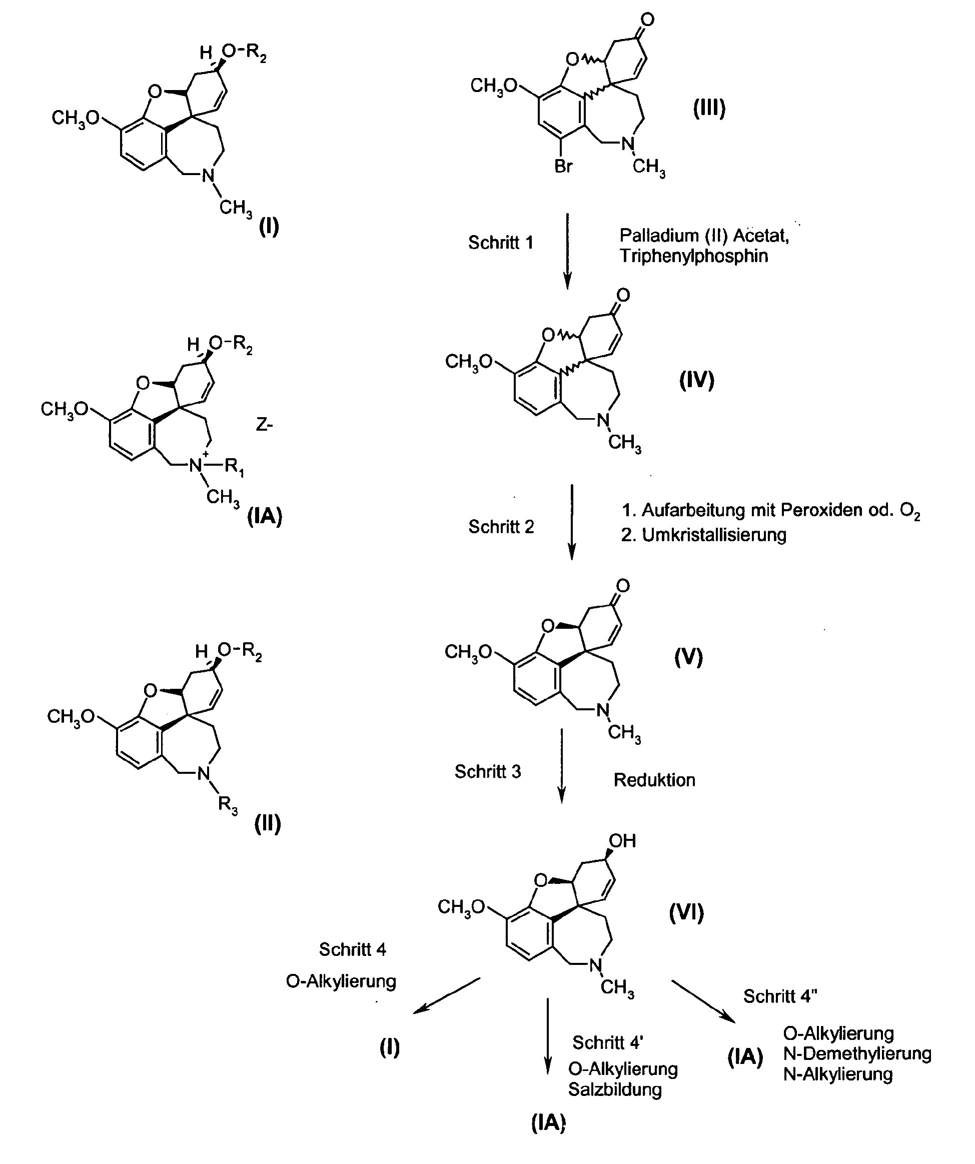

Vorteilhafte Ausgestaltungen der erfindungsgemäßen Verfahren sind Gegenstände der Unteransprüche.

Die Erfindung wird im Folgenden anhand von Ausführungsbeispielen zum Durchführen der Erfindung näher erläutert, wobei auf die Verfahrensschritte gemäß Reaktionsschema Bezug genommen wird:
Schritt 1: Racemisches Bromnarwedin der allgemeinen Formel (III) wird in DMF aufgenommen, mit NaCO2H, PPH3, Palladium(II)Acetat sowie Natriumhydroxid versetzt. Diese Reaktionsmischung wird auf 94°C erhitzt und sechs Stunden auf dieser Temperatur gehalten, wobei der Reaktionsverlauf mittels Chromatographie verfolgt wird. Anschließend wird das Reaktionsgemisch aufgearbeitet, wobei man DMF abdestilliert, das racemische Narwedin (IV) durch Zugabe von Wasser ausfällt und abtrennt.
Schritt 2.1: Das erhaltene recemische Narwedin (IV) wird in einem Gemisch von Ethanol/Triethylamin aufgenommen und mit Aktivkohle und einem Filterhilfsmittel versetzt. Die Mischung wird unter intensivem Rühren ein bis vier Stunden unter Rückfluss erhitzt, wobei ein Luft-Stickstoffgemisch mit beispielsweise 5 Vol.% Sauerstoff durch den Reaktor geblasen wird. Überraschender Weise wurde gefunden, dass durch die Behandlung mit Aktivkohle einerseits und den Sauerstoffkontakt anderseits die Reduktion der Palladiumanteile von deutlich über 95 % im Vergleich zu bekannten, nachweisbaren Palladiumanteilen erreicht werden konnte. Dies soll anhand folgender Tabelle näher erläutert werden:

| | 1. Charge | 2. Charge | 3. Charge |
|---|---|---|---|
| | Pd (ppm) | Pd (ppm) | Pd (ppm) |
| Racemisches Narwedin | 813 | 748 | 753 |
| (-)-Narwedin | 24 | 26 | 14 |

Aus dieser tabellarischen Aufstellung ist zu ersehen, dass im racemischen Narwedingemisch Palladiumreste von 748 bis 813 ppm nachweisbar sind. Reaktionsendprodukte mit diesen Anteilen an Palladium sind für eine weitere Verwendung für die Herstellung eines Arzneimittels ungeeignet. Durch die erfindungsgemäße Aufarbeitung des Reaktionsgemisches mit Aktivkohle bei gleichzeitigem Sauerstoffkontakt wird der Palladiumkatalysator in eine unlösliche, oxidierte Form übergeführt, sodass eine Abtrennung in einem ppm-Bereich von weniger als 26, bevorzugter Weise von weniger als 24, besonders bevorzugter Weise von weniger als 14 möglich ist.

In einer alternativen Verfahrensvariante wird das erhaltene racemische Narwedin (IV) ebenso in einem Gemisch von 30 Ethanol/Triethylamin aufgenommen und mit Aktivkohle und einem Filtrierhilfsmittel versetzt; allerdings wird diese Mischung anschließend unter intensivem Rühren mit 0,1 -1 Gew% Wasserstoffperoxid langsam versetzt und ein bis vier Stunden unter Rückfluss erhitzt. Überraschender Weise wurde auch bei dieser Verfahrensvariante gefunden, dass durch die Behandlung mit Aktivkohle einerseits und die Verwendung von Wasserstoffperoxid andererseits nach Filtration der Palladiumanteil im Vergleich zu bekannten, nachweisbaren Palladiumanteilen deutlich reduziert werden konnte. Die gemessenen Werte sind der folgenden Tabelle zu entnehmen:

| | 1.Charge | 2.Charge | 3.Charge |
|---|---|---|---|
| | Pd (ppm) | Pd (ppm) | Pd (ppm) |
| Racemisches Narwedine | 800 | 810 | 763 |
| (-)-Narwedine (H2O2 behandelt) | 22 | 24 | 16 |

In einer weiteren Verfahrensvariante wird die Mischung bestehend aus racemischem Narwedin (IV), Ethanol, Triethylamin, Aktivkohle und einem Filtrierhilfsmittel unter intensivem Rühren mit 0,1 -1 Gew% Metachlorperbenzosäure versetzt und ein bis vier Stunden unter Rückfluss erhitzt.
Auch bei dieser Verfahrensvariante wurde Überraschender Weise gefunden, dass durch die Behandlung mit Aktivkohle einerseits und die Verwendung von Metachlorperbenzosäure andererseits nach Filtration der Palladiumanteil im Vergleich zu bekannten, nachweisbaren Palladiumanteilen wesentlich reduziert werden konnte. Die ermittelten Werte sind in folgender Tabelle angeführt:

| | 1.Charge | 2.Charge | 3.Charge |
|---|---|---|---|
| | Pd (ppm) | Pd (ppm) | Pd (ppm) |
| Racemisches Narwedine | 778 | 805 | 767 |
| (-)-Narwedine (MCPBA behandelt) | 20 | 23 | 18 |

Schritt 2.2: Das nach Schritt 2.1 erhaltene Reaktionsgemisch wird gekühlt und mit (-) Narwedinkristallen angeimpft, sodass enantiomeren-reines (-)Narwedin mit der allgemeinen Formel (V) erhalten wird.
Schritt 3: Das nach dem Umkristallisieren erhaltene enantiomerenreine (-)Narwedin mit der allgemeinen Formel (V) wird, wie in der WO-A-96/12692 beschrieben, mit einer einmolaren 5 Lösung von L-Selektrid in THF versetzt, eine Stunde rühren gelassen, mit Ethanol versetzt und eingedampft. Durch die enantiomerselektive Reduktion wird enantiomerenreines Galanthamin der allgemeinen Formel (VI) erhalten. Durch ein- oder mehrmaliges Umkristallisieren werden Restanteile von Palladium von weniger als 5 ppm erzielt. Dies deshalb, da durch die Aufarbeitung mit Sauerstoff oder Peroxid gemäß Syntheseschritt 2.1 der Palladiumkatalysator in eine unlösliche, oxidierte Form übergeführt wird, welche sich im Zuge der Reinigung durch Umkristallisieren leicht abtrennen lässt.
Schritt 4: Die Verbindung der allgemeinen Formel (VI) kann einer O-Alkylierung unterworfen werden, um so die Reste R2 am Sauerstoff-Atom einzuführen.

### Beispiel: SPH-1313

10g Galanthamin werden in 100 ml Pyridin gelöst und Acetylchlorid bei 25 °C langsam zugeben. 5h bei RT rühren und 5h bei 50 °C rühren. Anschliessend das Pyridin abrotieren und den Rückstand in Wasser aufnehmen und mit Ethylacetat ausschütteln. Das organische Phase wird einrotiert und das Rohprodukt aus Ethanol umkristallisiert. Ausbeute 43,6 % Der gemessene Palladiumgehalt betrug < 5 ppm.

Schritt 4': Schritt 4' erfolgt analog zu Schritt 4 mit dem Unterschied, dass eine weitere Umsetzung mit einer Säure, wie beispielsweise Hydrobromid, zu pharmazeutisch akzeptablen Salzen mit Gegenanionen Z- wie beispielsweise ein Bromid, erfolgt.

Schritt 4": Die Verbindung der Formel (VI) kann einer N-Demethylierung unterzogen werden mit anschließender N- und O-Derivatisierung.

### Beispiel SPH-1297:

Zu einer Lösung von 1g Galanthamin in 50 ml Dichlormethan werden unter Schutzatmosphäre 1.0 ml Vinylchloroformiat und 1,2g 1,8-Bis(dimethylamino)naphtalin zugegeben. Das Reaktionsgemisch wird 18 h bei 65°C gerührt, das Lösungsmittel abdestilliert und das Rohprodukt aus Ethanol umkristallisiert.
Ausbeute 82,0 %
Der gemessene Palladiumgehalt betrug < 5 ppm

Die Verbindungen mit der allgemeinen Formel (I), (IA) oder (II) können, falls notwendig, weiter durch Umkristallisieren gereinigt werden, sodass ein Restanteil von weniger als 5 ppm erzielt wird. Die vorgenannten Ausführungsbeispiele wurden derart durchgeführt, dass R2 ein Substituentenmuster zeigt, worin R2 Carbonyl-, Carbonyloxygruppe und Carboxamid darstellt. Diese beispielhafte Auswahl ist allerdings nicht als Einschränkung des Schutzumfanges zu bewerten. Die pharmakologische Wirkung der Verbindungen gemäß der allgemeinen Formeln (I), (IA) und (II) lässt sich anhand der gemessenen IC50 Werte belegen, da diese jene Konzentrationen repräsentieren, bei welchen eine 50%-ige Hemmung der Acetylcholinesterase (AChEI) bzw. der Butyrylcholinesterase (BuCHEI) eintritt. Zufriedenstellende Hemmwerte - siehe folgende Übersicht - sind des Weiteren ein Indiz dafür, dass die Verbindungen der allgemeinen Formeln (I) (IA) bzw. (II) zur Herstellung von Arzneimitteln für die Behandlung von neurodegenerativen Erkrankungen, wie die Alzheimer'sche Erkrankung, geeignet sind.

**Tabelle 1: Beispiele für Verbindungen der allgemeinen Formeln (I), (IA) und (II) und Ergebnisse der Acetylcholinesterase- und Butylcholinesterasehemmung**

| **SPH** | **STRUCTURE** | **AChE final** | **BChE final** | **Typ** |
|---|---|---|---|---|
| SPH-1001 | 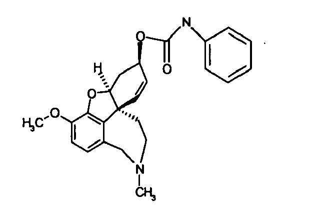 | 200 | 200 | I |
| SPH-1002 | 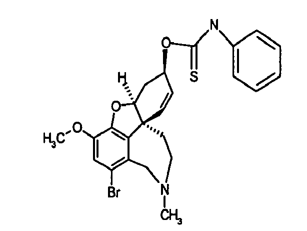 | 45 | 52 | I |
| SPH-1003 | 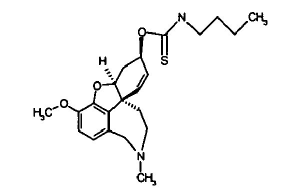 | 200 | 3,8 | I |
| SPH-1005 | 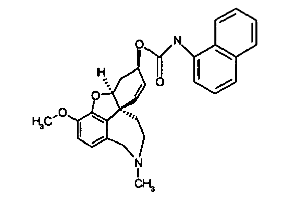 | 200 | 200 | I |
| SPH-1006 | 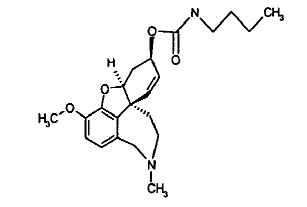 | 200 | 200 | I |
| SPH-1007 | 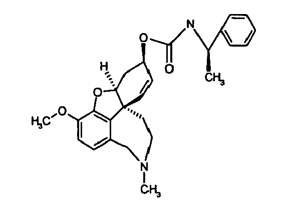 | 200 | 50 | I |
| SPH-1008 | 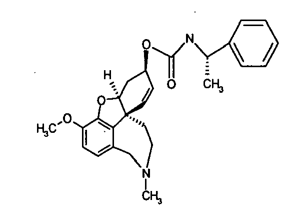 | 94 | 77 | I |
| SPH-1010 | 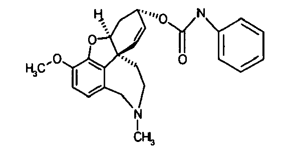 | 90 | 200 | I |
| SPH-1011 | 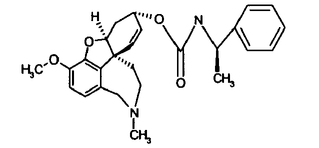 | 75 | 40 | I |
| SPH-1012 | 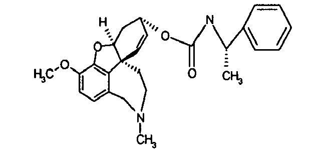 | 70 | 80 | I |
| SPH-1013 | 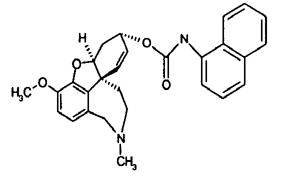 | 200 | 200 | I |
| SPH-1014 | 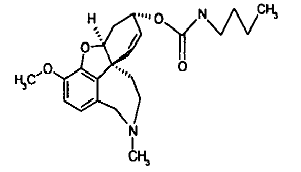 | 200 | 200 | I |
| SPH-1015 | 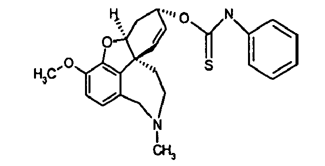 | 30 | 15 | I |
| SPH-1016 | 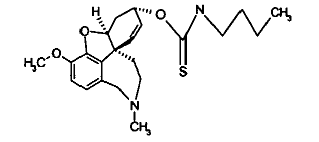 | 40 | 20 | I |
| SPH-1022 | 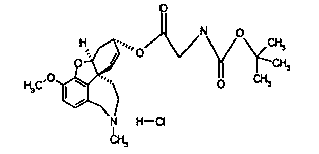 | 200 | 50 | IA |
| SPH-1025 | 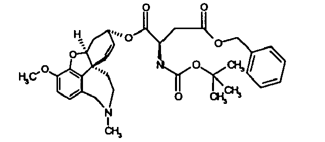 | 18 | 4 | I |
| SPH-1026 | 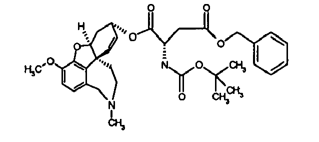 | 11 | 115 | I |
| SPH-1035 | 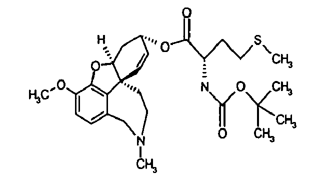 | 4 | 171 | I |
| SPH-1036 | 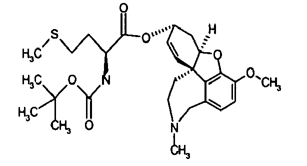 | 16 | 140 | I |
| SPH-1037 | 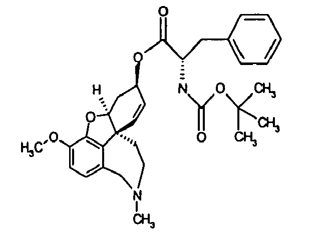 | 19 | 172 | I |
| SPH-1039 | 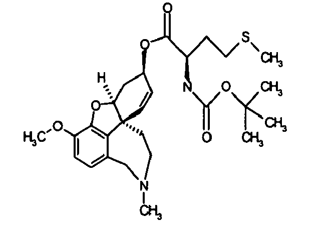 | 15 | 42 | I |
| SPH-1043 | 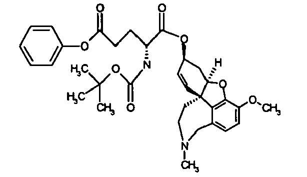 | 19 | 6 | I |
| SPH-1137 | 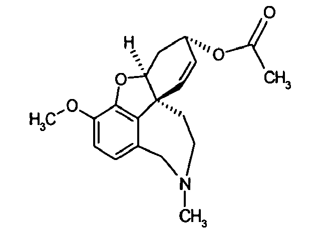 | 200 | 200 | I |
| SPH-1297 | 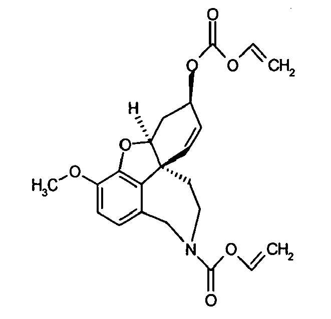 | 200 | 200 | II |
| SPH-1313 | 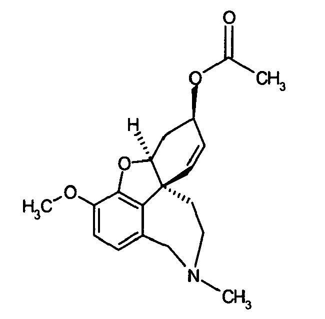 | 31 | 200 | I |
| SPH-1351 | 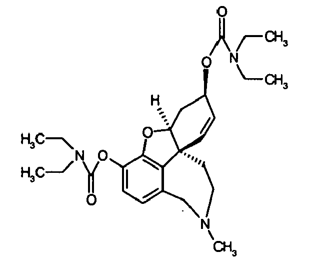 | 11,1 | 16,7 | I |
| SPH-1370 | 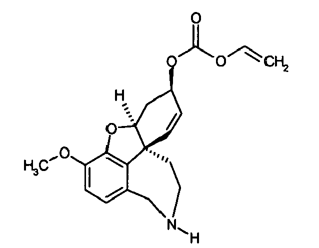 | 17 | 21 | II |
| SPH-1391 | 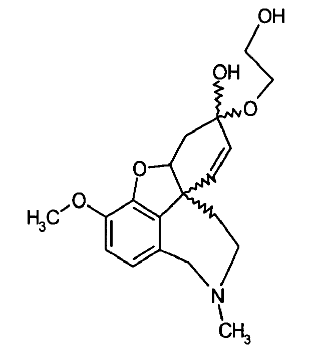 | 18 | 195 | I |
| SPH-1396 | 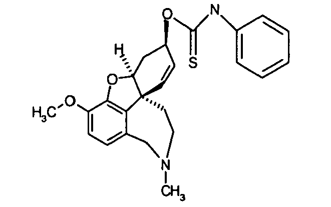 | 51 | 30 | I |
| SPH-1397 | 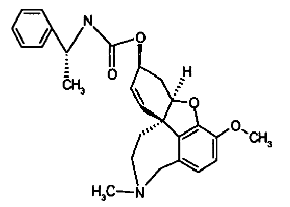 | 10 | 53 | I |
| SPH-1398 | 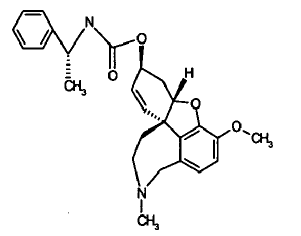 | 16 | 154 | I |
| SPH-1399 | 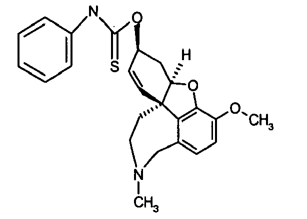 | 19 | 32 | I |
| SPH-1400 | 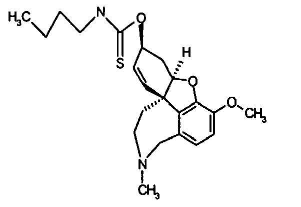 | 19 | 93 | I |
| SPH-1401 | 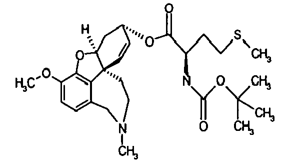 | 10 | 130 | I |
| SPH-1402 | 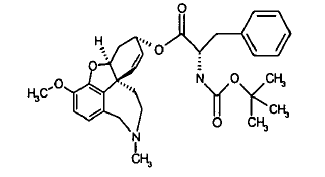 | 16 | 51 | I |
| SPH-1403 | 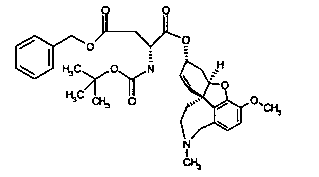 | 6 | 175 | I |
| SPH-1404 | 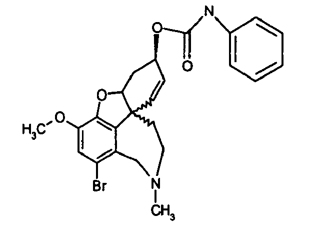 | 7 | 33 | I |
| SPH-1405 | 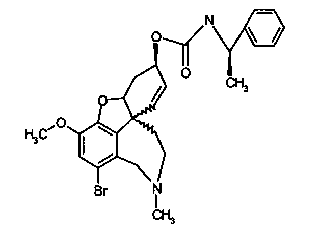 | 5 | 31 | I |
| SPH-1524 | 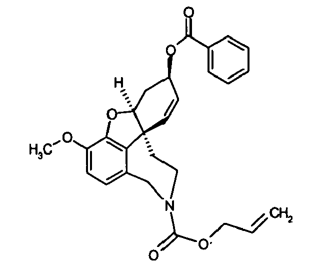 | 1 | 97 | II |
| SPH-1526 | 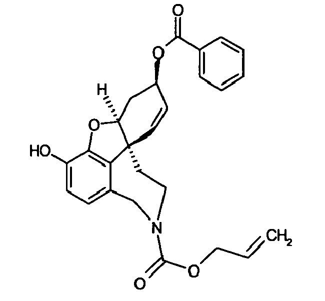 | 11 | 120 | II |
| SPH-1538 | 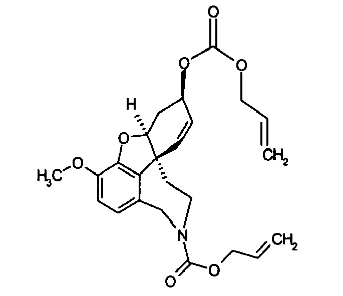 | 1 | 110 | II |
| SPH-1541 | 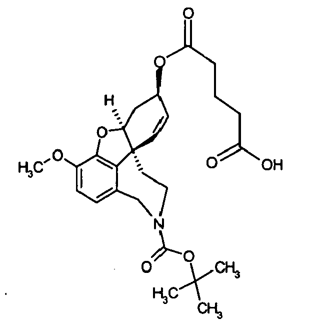 | 0 | 53 | II |
| SPH-1542 | 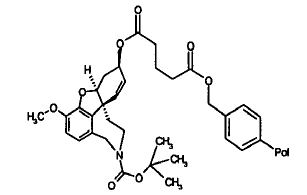 | 8 | 88 | II |
| SPH-3272 | 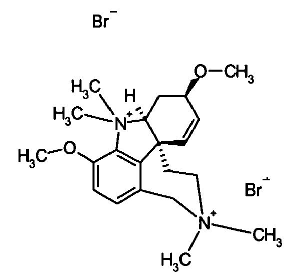 | 18 | 194 | IA |
| SPH-3283 | 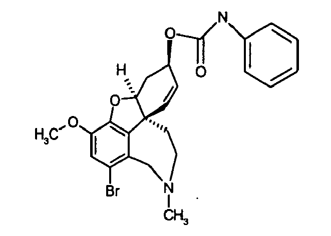 | 18 | 194 | I |
| SPH-3284 | 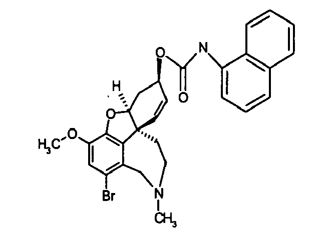 | 4 | 90 | I |
| SPH-3285 | 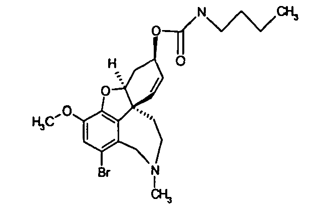 | 2 | 39 | I |
| SPH-3298 | 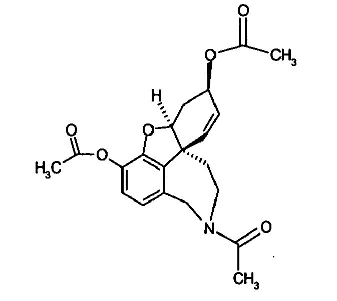 | 12 | 151 | II |
| SPH-3364 | 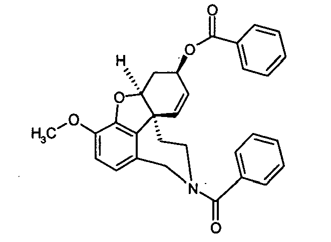 | 16 | 158 | II |
| SPH-3366 | 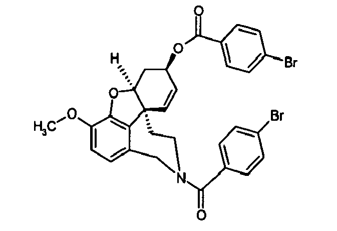 | 19 | 69 | II |
| SPH-3417 | 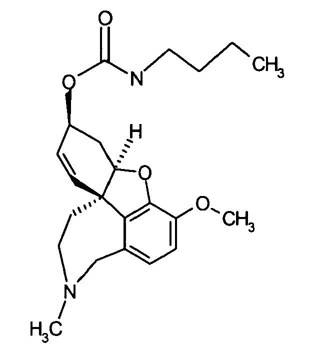 | 200 | 200 | I |

Der Substituent R₂ in der allgemeinen Formel (I), (IA) und (II) kann abgesehen von den eingangs genannten, bevorzugten Bedeutungen auch bedeuten:
i) Wasserstoff, eine niedrige (C₁-C₁₀, gegebenenfalls verzweigte oder substituierte) Alkylgruppe, oder Cycloalkylgruppe, eine C₃-C₁₀ substituierte Silylgruppe (beispielsweise Triethylsilyl, Trimethylsilyl, t-Butyldimethylsilyl oder Dimethylphenylsilyl), eine C₂-C₁₀-alpha-Alkoxyalkyl-Gruppe, beispielsweise Tetrahydropyranyl, Tetrahydrofuranyl, Methoxymethyl, Ethoxymethyl, 2-Metho-xypropyl, Ethoxyethyl, Phenoxymethyl oder 1-Phenoxyethyl;
ii) O-CS-NHR₆ (Thiourethane), worin R₆ die oben unter i) angegebene Bedeutungen hat;
iii) O-CO-NHR₇ mit der nachstehenden Bedeutung: 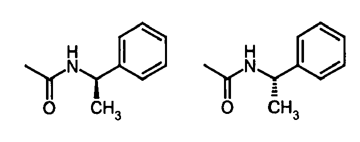
iv) O-CO-HR₆, worin R₆ die oben unter i) genannte Bedeutungen hat, insbesondere Ester mit den Substitutionsmuster von Aminosäuren (beide Enantiomeren), wie

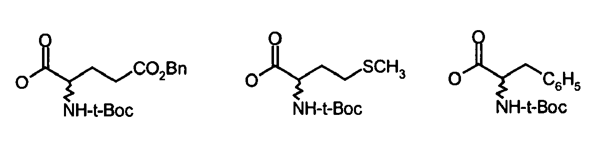

Zusammenfassend kann gesagt werden, dass durch die erfindungsgemäße Aufarbeitung eines durch Palladiumkatalyse gewonnenen debromierten Narwedin, nämlich durch Kontakt mit Sauerstoff oder Peroxiden, der eingesetzte Palladiumkatalysator in eine unlösliche Oxidform übergeführt und in einfacher Weise abgetrennt werden kann. Durch diese den Sicherheitsvorschriften durchaus gerecht werdende Aufarbeitung des Reaktionsgemisches gelang es in überraschender Weise die Palladiumreste unterhalb von 5 ppm zu reduzieren, sodass hochreines Galanthamin bzw. hochreine Galanthaminderivate gewonnen werden konnten, welche(s) unmittelbar in die Herstellung von Arzneimitteln, wie beispielsweise solche für die Behandlung der Alzheimer'schen Erkrankung, eingesetzt werden konnten (kann).

Die Verbindungen, die gemäß der Erfindung erhältlich sind, sowie pharmazeutisch annehmbare Säureadditionssalze derselben können Wirkstoffe von Arzneimitteln zur Behandlung von neurodegenerativen Prozessen dienen, wobei insbesondere nicht vorrangig auf eine Verbesserung der akuten Symptomatik, sondern auf eine Verlangsamung und Modifizierung der damit verbundenen Prozesse abgezielt wird.

Im Rahmen des Diabetes mellitus Typ II findet sich zunehmend Evidenz für eine Rolle von Amyloid Fragmenten bei der Zelldegeneration der Insulin-produzierenden Langerhans'schen Inselzellen. Über einen nicht-kontrollierten Calciumionenstrom kann die Zelldegeneration verstärkt werden.

Die Verbindungen, die gemäß der Erfindung erhältlich sind, sowie pharmazeutisch annehmbaren Säureadditionssalze derselben können als Wirkstoffe in Arzneimitteln beispielsweise zum Behandeln von degenerativen Erkrankungen der Inselzellen (wie z.B. Diabetes mellitus Typ II) eingesetzt werden.

Die Verbindungen, die gemäß der Erfindung erhältlich sind, können als Wirkstoffe in Arzneimitteln verwendet werden, die wie folgt eingesetzt werden können:
a) zur Behandlung der Alzheimer'schen Krankheit,
b) zur Behandlung der Parkinson'schen Krankheit,
c) zur Behandlung der Huntington'schen Krankheit (Chorea),
d) zur Behandlung der Multiplen Sklerose,
e) zur Behandlung der Amyotrophen Lateralsklerose,
f) zur Behandlung der Epilepsie,
g) zur Behandlung der Folgen des Schlaganfalles,
h) zur Behandlung der Folgen des Schädel-Hirn-Traumas,
i) zur Behandlung und Prophylaxe der Folgen diffusen Sauerstoff- und Nährstoffmangels im Gehirn, wie sie nach Hypoxie, Anoxie, Asphyxie, Herzstillstand, Vergiftungen, sowie bei Komplikationen bei schweren Geburten am Säugling oder bei Narkosen beobachtet werden,
j) zur insbesondere auch prophylaktischen Behandlung apoptotischer Degeneration in Neuronen, die durch lokale Radio- oder Chemotherapie von Gehirntumoren geschädigt wurden bzw. werden, und
k) zur Behandlung der bakteriellen Meningitis und
l) zur Behandlung von Erkrankungen mit apoptotischer Komponente, besonders im Gefolge von amyloid-assozüerter Zelldegeneration
m) zur Behandlung des Diabetes mellitus, insbesondere, wenn er mit Amyloiddegeneration der Inselzellen einhergeht.
n) zum Erhöhen der Muskelkraft und der Ausdauer von Alzheimer-Patienten

Die erfindungsgemäß erhältlichen Verbindungen oder deren pharmazeutisch annehmbare Säureadditionssalze, z.B. Hydrobromid, Hydrochlorid, Methylsulfat, Methiodid, Tartrat, Fumarat, Oxalat etc. (siehe nachstehende Tabelle), können Patienten oral, rektal oder durch subkutane, intramuskuläre, intravenöse oder intrathekale Injektion oder Infusion, oder intracerebroventrikulär, z.B. mittels eines implantierten Behälters verabreicht werden.

Beispiele für in Betracht gezogene Salze erfindungsgemäß erhältlicher Verbindungen, sind in der nachstehenden Tabelle angeführt:

| englisch | Säure | Salz |
|---|---|---|
| | | |
| Sulfamic | Sulfaminsäure Amidosulfonsäure | - Amidosulfonat |
| 1,2-ethanedisulfonic | 1,2-Ethandisulfonsäure | 1,2-Ethandisulfonat |
| 2-Ethylsuccinic | 2-Ethylbernsteinsäure | 2-Ethylsuccinat |
| 2-hydroxyethanesulfonic= isethionic | 2-Hydroxyethansulfonsäure | 2-Hydroxyethansulfonat |
| 3-Hydroxynaphthoic | 3-Hydroxynaphthoesäure | 3-Hydroxynaphthoat |
| acetic | Essigsäure | Acetat |
| benzoic | Benzoesäure | Benzoat |
| benzenesulfonic | Benzolsulfonsäure | Benzolsulfonat |
| calcium dihydrogenedetic | Calciumdihydrogenethylendiamintetraessigsäure | Calciumethylendiamintetraacetat |
| camphorsulfonic | Camphersulfonsäure | Camphersulfonat |
| carbonic | Kohlensäure | Carbonat |
| citric | Zitronensäure | Citrat |
| Dodecylsulfonic | Dodecylsulfonsäure | Dodecylsulfonat |
| ethanesulfonic | Ethansulfonsäure | Ethansulfonat |
| edetic | Ethylendiamintetraessigsäure | Ethylendiamintetraacetat |
| fumaric | Fumarsäure | Fumarat |
| Glubionic | Glubionsäure | Glubionat |
| glucoheptonic | Glucoheptonsäure | Glucoheptonat |
| gluconic | Gluconsäure | Gluconat |
| glutamic | Glutaminsäure | Glutamat |
| hexylresorcinic | Hexylresorcylsäure | Hexylresorcylat |
| HBr | Bromwasserstoffsäure | Hydrobromid |
| HCl | Salzsäure | Hydrochlorid |
| bicarbonic | Kohlensäure | Hydrogencarbonat |
| englisch | Säure | Salz |
| bitartaric | Weinsäure | Hydrogentartrat |
| hydriodic | lodwasserstoffsäure | Hydroiodid |
| lactic | Milchsäure | Lactat |
| lactobionic | Lactobionsäure | Lactobionat |
| Levulinic | Laevulinsäure | Laevulinat |
| estolic (laurylsulfuric) | Laurylschwefelsäure | Laurylsulfat |
| LIPOIC-(ALPHA) ACID | Liponsäure | Liponat |
| malic | Äpfelsäure | Malat |
| maleic | Maleinsäure | Maleinat |
| Malonic | Malonsäure | Malonat |
| methanesulfonic | Methansulfonsäure | Methansulfonat |
| naphthalenesulfonic | Napthalinsulfonsäure | Napthalinsulfonat |
| nitric | Salpetersäure | Nitrat |
| Pantothenic | Pantothensäure | Pantothenat |
| phosphoric | Phosphosäure | Phosphat |
| polygalacturonic | Polygalacturonsäure Pectinsäure | Polygalacturonat |
| Propionic | Propionsäure | Propionat |
| salicylic | Salicylsäure | Salicylat |
| succinic | Bernsteinsäure | Succinat |
| sulfuric | Schwefelsäure | Sulfat |
| Tartaric | Weinsäure | Tartrat |

Typische Dosierungsraten bei Verabreichung erfindungsgemäß erhaltener Verbindungen als Wirkstoffe hängen von der Natur der verwendeten Verbindung ab und liegen bei intravenöser Applikation im Bereich von 0,01 bis 2,0 mg pro Tag und Kilogramm Körpergewicht in Abhängigkeit vom physischen Zustand und sonstiger Medikation des Patienten.

Die folgenden spezifischen Formulierungen können Anwendung finden:
Tabletten und Kapseln enthaltend 0,5 bis 50 mg
Lösung zur parenteralen Verabreichung enthaltend 0,1 bis 30 mg Wirkstoff/ml
flüssige Formulierungen zur oralen Verabreichung in einer Konzentration von 0,1 bis 15 mg/ml
flüssige Formulierungen zur intracerebroventrikulären Verabreichung, in einer Konzentration von 1 oder 5 mg Wirkstoff/ml.

Die Verbindungen können auch ein transdermales System sein, in welchem 0,1 bis 10 mg/Tag freigesetzt werden.

Ein transdermales Dosiersystem besteht aus einer Vorratsschicht, welche 0,1 bis 30 mg der Wirksubstanz als freie Base oder Salz allenfalls zusammen mit einem Penetrationsbeschleuniger, z.B. Dimethylsulfoxid oder einer Carbonsäure, z.B. Octansäure, und einem hautnahen Polyacrylat, z.B. HexylacrylatNinylacetat/Acrylsäure Copolymer samt Weichmacher, z.B. Isopropylmyristat enthält. Als Abdeckung dient eine wirkstoffundurchlässige Außenschicht, z.B. ein metallbeschichtetes, siliconisiertes Polyethylenpflaster mit einer Dicke von beispielsweise 0,35 mm. Zur Erzeugung einer klebenden Schicht dient z.B. ein Dimethylaminomethycrylat/Methacrylat Copolymer in einem organischen Lösungsmittel.

Insbesondere sind die erfindungsgemäß erhaltener Verbindungen, die vielfach eine die Cholinesterase hemmende Wirkung zeigen, als therapeutische und/oder prophylaktische Wirkstoffe für die senile Demenz, Alzheimer-Krankheit, usw. geeignet. Die erfindungsgemäß erhältliche Verbindungen sind neue, hochreine Formen tetrazyklischer, kondensierter, heterocyclischer Verbindungen.

Zusammenfassend kann ein Ausführungsbeispiel der Erfindung wie folgt dargestellt werden:

Die Erfindung betrifft ein Verfahren zur Herstellung von hochreinem Galanthamin bzw. hochreinen Galanthaminderivaten, wobei man von racemischem Bromnarwedin ausgeht, welches unter Palladiumkatalyse debromiert wird. Erfindungswesentlich ist dabei die Aufarbeitung des Reaktionsgemisches, welche in Gegenwart von Sauerstoff oder Peroxiden erfolgt, sodass der Palladiumkatalysator in eine unlösliche Form, leicht abtrennbare Form übergeführt wird. Die weitere Reaktion erfolgt durch Reduktion von enantiomerenreinem Narwedin zu enantiomerenreinem Galanthamin, wobei anschließend alkyliert bzw. dealkyliert wird, sodass eine entsprechende Substitution am RingStickstoffatom erzielt wird. Durch weitere Reinigung, wie Umkristallisieren, werden Restanteile von Palladium unterhalb von 5 ppm erzielt, sodass die direkte Verwendung als pharmazeutischer Rohstoff ermöglicht wird.

## Patentansprüche

1. Verfahren zum Herstellen von hochreinen 4a,5,9,10,11,12,-Hexahydro-6H-benzofuro[3a,3,2-ef][2]benzazepinderivaten mit der allgemeinen Formel I, IA und II: 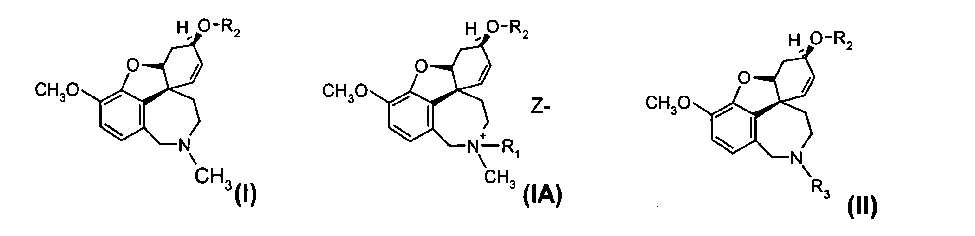 worin R1 ausgewählt ist aus der Gruppe bestehend aus Wasserstoff, Hydroxy, Alkoxy, niedriges Alkyl (C2-C10), welches gegebenenfalls durch wenigstens ein Halogen substituiert ist, niedriges Alkenyl (C2-C10), Aryl, Aralkyl, Aryloxyalkyl, R2 ausgewählt ist aus der Gruppe bestehend aus niedriges Alky (C2-C10)I, welches gegebenenfalls durch wenigstens ein Halogen substituiert ist, niedriges Alkenyl (C2-C10), niedriges Alkinyl (C2-C10), Aryl, Aralkyl, Aryloxyalkyl, Alkylcarbonyl, Arylcarbonyl, Aralkylcarbonyl, Alkyloxycarbonyl, Aryloxycarbonyl, Aralkyloxycarbonyl, Alkylthionyl, Arylthionyl, Aralkylthionyl, Alkyloxythionyl, Aryloxythionyl, Aralkyloxythionyl, Alkylsulfonyl, Aralkylsulfonyl, Arylsulfonyl, Carboxamid, Thiocarboxamid R3 ausgewählt ist aus der Gruppe bestehend aus Wasserstoff, Hydroxy, Alkoxy, niedriges Alkyl (C2-C10), welches gegebenenfalls durch wenigstens ein Halogen substituiert ist, niedriges Alkenyl (C2-C10), niedriges Alkinyl (C2-C10), Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl und wobei Z-ein Anion einer pharmazeutisch annehmbaren organischen Säure oder ein anorganisches Anion ist, **dadurch gekennzeichnet, dass** man in einem Reaktionsschritt 1 racemisches Bromnarwedin (III) mit Palladium(II)Acetat und Triphenylphosphin debromiert, in einem Reaktionsschritt 2 das Reaktionsgemisch, beinhaltend racemisches Narwedin (IV) unter Sauerstoffkontakt aufarbeitet und in einem zum enantiomerenreinem Narwedin (V) umwandelt und wobei man in einem Reaktionsschritt 3 durch Reduktion enantiomerenreines Galanthamin der allgemeinen Formel (VI) erhält und in einem Reaktionsschritt 4 durch O-Alkylierung Verbindungen der allgemeinen Formel (I) bzw. in einem Reaktionsschritt 4' durch O-Alyklierung sowie anschließender Salzbildung Verbindungen der allgemeinen Formel (IA) erhält bzw. in einem Raktionsschritt 4" durch O-Alyklierung, N-Demethylierung und N-Alkylierung Verbindungen der allgemeinen Formel (II) erhält.

2. Verfahren nach Anspruch 1, **dadurch gekennzeichnet, dass** der Sauerstoffkontakt im Reaktionsschritt 2 mit einem Luft-Stickstoff-Gemisch erfolgt.

3. Verfahren nach Anspruch 2, **dadurch gekennzeichnet, dass** das Luft-Stickstoff-Gemisch 0,2 bis 20 Vol% Sauerstoff enthält.

4. Verfahren nach einem der Ansprüche 1 bis 3, **dadurch gekennzeichnet, dass** der Sauerstoffkontakt in Gegenwart von Aktivkohle erfolgt.

5. Verfahren nach einem der Ansprüche 1 bis 4, **dadurch gekennzeichnet, dass** dem Reaktionsschritt 3 und/ oder dem Reaktionsschritt 4 ein oder mehrere Reinigungsschritt(e), vorzugsweise Umkristallisieren, nachgeschaltet sind (ist).

6. Verfahren zum Herstellen von hochreinem 4a,5,9,10,11,12,-Hexahydro-6H-benzofuro[3a,3,2-ef][2]benzazepinderivaten mit der allgemeinen Formel I, IA und II 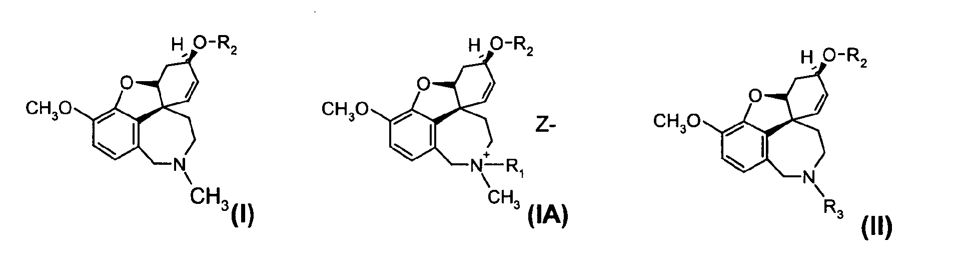 worin R1 ausgewählt ist aus der Gruppe bestehend aus Wasserstoff, Hydroxy, Alkoxy, niedriges Alkyl (C2-C10), welches gegebenenfalls durch wenigstens ein Halogen substituiert ist, niedriges Alkenyl (C2-C10), Aryl, Aralkyl, Aryloxyalkyl, R2 ausgewählt ist aus der Gruppe bestehend aus niedriges Alky (C2-C10)I, welches gegebenenfalls durch wenigstens ein Halogen substituiert ist, niedriges Alkenyl (C2-C10), niedriges Alkinyl (C2-C10), Aryl, Aralkyl, Aryloxyalkyl, Alkylcarbonyl, Arylcarbonyl, Aralkylcarbonyl, Alkyloxycarbonyl, Aryloxycarbonyl, Aralkyloxycarbonyl, Alkylthionyl, Arylthionyl, Aralkylthionyl, Alkyloxythionyl, Aryloxythionyl, Aralkyloxythionyl, Alkylsulfonyl, Aralkylsulfonyl, Arylsulfonyl, Carboxamid, Thiocarboxamid R3 ausgewählt ist aus der Gruppe bestehend aus Wasserstoff, Hydroxy, Alkoxy, niedriges Alkyl (C2-C10), welches gegebenenfalls durch wenigstens ein Halogen substituiert ist, niedriges Alkenyl (C2-C10), niedriges Alkinyl (C2-C10), Aryl, Aralkyl, Aryloxyalkyl, Formyl, Alkylcarbonyl, Arylcarbonyl, Aralkylcarbonyl, Alkyloxycarbonyl, Aryloxycarbonyl, Aralkyloxycarbonyl, Alkylsulfonyl, Aralkylsulfonyl, Arylsulfonyl und wobei Z-ein Anion einer pharmazeutisch annehmbaren organischen Säure oder ein anorganisches Anion ist, **dadurch gekennzeichnet, dass** man in einem Reaktionsschritt 1 racemisches Bromnarwedin (III) mit Palladium(II)Acetat und Triphenylphosphin debromiert, in einem Reaktionsschritt 2 das Reaktionsgemisch, beinhaltend racemisches Narwedin (IV) in Gegenwart von Peroxiden aufarbeitet und in einem zum enantiomerenreinem Narwedin (V) umwandelt und wobei man in einem Reaktionsschritt 3 durch Reduktion enantiomerenreines Galanthamin der allgemeinen Formel (VI) erhält und in einem Reaktionsschritt 4 durch O-Alkylierung Verbindungen der allgemeinen Formel (I) bzw. in einem Reaktionsschritt 4' durch O-Alyklierung sowie anschließender Salzbildung Verbindungen der allgemeinen Formel (IA) erhält bzw. in einem Reaktionsschritt 4" durch O-Alyklierung, N-Demethylierung und N-Alkylierung Verbindungen der allgemeinen Formel (II) erhält.

7. Verfahren nach Anspruch 6, **dadurch gekennzeichnet, dass** im Reaktionsschritt 2 als Peroxide anorganische Peroxide, vorzugsweise Wasserstoffperoxid eingesetzt werden.

8. Verfahren nach Anspruch 6, **dadurch gekennzeichnet, dass** im Reaktionsschritt 2 als Peroxide organische Peroxide, vorzugsweise Metachlorperbenzoesäure eingesetzt werden.

9. Verfahren nach einem der Ansprüche 6 bis 8 **dadurch gekennzeichnet, dass** im Reaktionsschritt 2 neben den Peroxiden auch Aktivkohle vorliegt.

10. Verfahren nach einem der Ansprüche 6 bis 9, **dadurch gekennzeichnet, dass** dem Reaktionsschritt 3 und/ oder dem Reaktionsschritt 4 ein oder mehrere Reinigungsschritt(e), vorzugsweise Umkristallisieren, nachgeschaltet sind (ist).
